# EUROPÄISCHE PATENTANMELDUNG

(11) **EP 4 591 811 A1**
(43) Veröffentlichungstag der Anmeldung: **30.07.2025**
(21) Anmeldenummer: 25153516.7
(22) Anmeldetag: 23.01.2025
(51) Int. Cl.: A61B 17/54, A61M 35/00, B65D 35/36, A45D 34/04, A61B 17/32

(54) **DERMABRASIONSWERKZEUG MIT AUSPRESSBAREM FLUIDBEHÄLTER**

(30) Priorität: 23.01.2024 DE 102024101897
(71) Anmelder: Bauer, Gwenaël, 67000 Strasbourg (FR)
(72) Erfinder: Lippert, Torsten Herbert, 77656 Offenburg (DE)
(74) Vertreter: Kohler Schmid Möbus Patentanwälte

(57) **Zusammenfassung**

Die Erfindung betrifft ein mit Fluid (20) gefülltes Dermabrasionswerkzeug (10) mit einer Öffnung (22) zum Austritt des Fluids (20). Ein Handteil (12) ist vorzugsweise zumindest teilweise containerartig oder tubenartig ausgebildet. Ein Dermabrasionsbereich (14) ist bevorzugt im Bereich der Öffnung (22) angeordnet oder ausgebildet, kann aber auch auf einer anderen Stelle des Dermabrasionsbereiches (14) liegen.

## Beschreibung

### Hintergrund der Erfindung

Die Erfindung betrifft ein Dermabrasionswerkzeug mit einem Handteil zum manuellen Halten und Führen des Dermabrasionswerkzeugs und einem Dermabrasionsbereich am axialen Ende des Dermabrasionswerkzeugs, wobei der Dermabrasionsbereich eine Trägermatrix mit mehreren Reibvorsprünge zur mechanischen Hautbehandlung aufweist, die an der Trägermatrix angeordnet oder in der Trägermatrix ausgebildet sind, wobei die Reibvorsprünge jeweils eine maximale Querschnittsbreite zwischen 0,5 µm und 1000 µm aufweisen, wobei das Handteil einen mit einem Fluid gefüllten Hohlraum aufweist, wobei das Dermabrasionswerkzeug eine mit dem Hohlraum fluidisch verbundene Öffnung aufweist.

Ein solches Dermabrasionswerkzeug ist aus der EP 3 165 124 B1 bekannt geworden. Das bekannte Dermabrasionswerkzeug weist einen Dermabrasionsbereich mit einer Reibfläche auf, um einen Hautbereich aufrauen zu können. Der aufgeraute Hautbereich kann mit dem Fluid behandelt werden.

Nachteilig an dem bekannten Dermabrasionswerkzeug ist dessen konstruktiv eher aufwendige Gestaltung und die damit einhergehenden hohen Produktionskosten zur Dosierung des jeweiligen Fluids.

### Aufgabe der Erfindung

Es ist daher Aufgabe der Erfindung, ein Dermabrasionswerkzeug bereitzustellen, dass signifikant kostengünstiger herstellbar ist, ohne die Vorteile des bekannten Dermabrasionswerkzeugs aufzugeben, und die gezielte und standardisierte Anwendung des Dermabrasionsbereichs zusammen mit dem Fluid zu vereinfachen.

### Beschreibung der Erfindung

Diese Aufgabe wird erfindungsgemäß gelöst durch ein Dermabrasionswerkzeug gemäß Patentanspruch 1. Die abhängigen Patentansprüche geben bevorzugte Weiterbildungen wieder.

Die erfindungsgemäße Aufgabe wird somit gelöst durch ein eingangs beschriebenes Dermabrasionswerkzeug, wobei das Handteil zumindest abschnittsweise elastisch und/oder plastisch verformbar ausgebildet ist, sodass ein Nutzer durch Pressen und/oder Drehen des Handteils das Fluid portionsweise aus der Öffnung pressen kann. Ein Nutzer kann schlicht durch Pressen und/oder Drehen des elastischen und/oder plastischen Abschnitts das Fluid aus der Öffnung drücken.

Unter dem zumindest abschnittsweise verformbaren Handteil wird erfindungsgemäß auch ein Pumpteil verstanden, wie es beispielsweise aus Spendern für Flüssigseife bekannt geworden ist.

Vorzugsweise weisen die Partikel eine maximale Querschnittsbreite zwischen 1 µm und 0,8 mm, insbesondere zwischen 1 µm und 350 µm, vorzugsweise zwischen 1 µm und 20 µm, auf.

Die Applikationsfläche des Dermabrasionsbereiches ist vorzugsweise rund oder oval oder linear und weist einen Durchmesser bzw. maximale Erstreckung zwischen 1 cm und 5 cm auf.

Das Fluid kann eine Säure, eine Lauge, eine Emulsion / Creme und/oder ein Medikament aufweisen. Das Fluid kann ein Pathogen aufweisen. In diesem Fall können durch das Eindringen mittels Mikroverletzungen einfacher Immunisierungen und/oder Sensibilisierungen (bei Allergikern) vorgenommen werden und/oderTherapeutika verabreicht werden.

Die zumindest abschnittsweise Elastizität und/oder Plastizität des Dermabrasionswerkzeugs kann durch ein Handteil herbeigeführt werden, welches eine Folie, insbesondere eine Kunststofffolie, aufweist.

Das Handteil ist vorzugsweise überwiegend elastisch, besonders bevorzugt vollständig elastisch, ausgebildet. Hierdurch kann das Fluid besonders einfach aus dem Dermabrasionswerkzeug gepresst werden.

Das Dermabrasionswerkzeug kann konstruktiv besonders einfach ausgebildet sein, wenn die Öffnung im Dermabrasionsbereich ausgebildet ist. Vorzugsweise ist die Öffnung radial vollständig vom Dermabrasionsbereich umringt oder befindet sich neben der Öffnung.

In besonders bevorzugter Ausgestaltung der Erfindung ist das Handteil in Form einer Tube oder eines Containers ausgebildet. Hierdurch wird die Fertigung und die Anwendung des Dermabrasionswerkzeugs signifikant vereinfacht.

Der Dermabrasionsbereich kann auf einem eigenständigen Bauteil angeordnet oder ausgebildet sein, das vom Handteil abnehmbar ist. Das Handteil inklusive des Fluids kann dadurch mit Abnahme des Dermabrasionsbereiches bzw. des Bauteils ausgewechselt werden, sodass der Dermabrasionsbereich auf einem neuen Handteil aufgebracht werden kann. Das Bauteil kann auf das Handteil aufgesteckt oder mittels einer Gewindeverbindung aufgeschraubt sein.

Das Dermabrasionswerkzeug kann einen Deckel zum Verschließen der Öffnung aufweisen.

Der zuvor bezeichnete Deckel kann in Form eines Schraubdeckels mit einem Deckelgewinde ausgebildet sein. Das Handteil kann einen axialen Gewindevorsprung mit einem zum Deckelgewinde passenden Handteilgewinde aufweisen. Weiter bevorzugt kann der Gewindevorsprung die Öffnung aufweisen.

In weiter bevorzugter Ausgestaltung dieser Ausführungsform kann der Gewindevorsprung den Dermabrasionsbereich aufweisen.

Das abnehmbare Bauteil kann den Gewindevorsprung aufweisen.

Die Fertigung des Dermabrasionswerkzeugs kann weiter vereinfacht werden, wenn die Trägermatrix in Form einer flachen Scheibe ausgebildet ist.

Weiter bevorzugt können die Reibvorsprünge in Form von Partikeln ausgebildet sein, die in die Trägermatrix eingebettet sind. Die Trägermatrix kann aus Glas, Kunststoff oder Papier ausgebildet sein. Die Partikel können in die Trägermatrix eingebrannt oder aufgebracht sein. Die Partikel können vorzugsweise in Form von Sandkörnern, Diamantstaub, Glaspartikeln, Metallpartikeln und/oder Kunststoffpartikeln ausgebildet sein.

Weitere Vorteile der Erfindung ergeben sich aus der Beschreibung und der Zeichnung. Ebenso können die vorstehend genannten und die noch weiter ausgeführten Merkmale erfindungsgemäß jeweils einzeln für sich oder zu mehreren in beliebigen Kombinationen Verwendung finden. Die gezeigten und beschriebenen Ausführungsformen sind nicht als abschließende Aufzählung zu verstehen, sondern haben vielmehr beispielhaften Charakter für die Schilderung der Erfindung.

### Detaillierte Beschreibung der Erfindung und Zeichnung

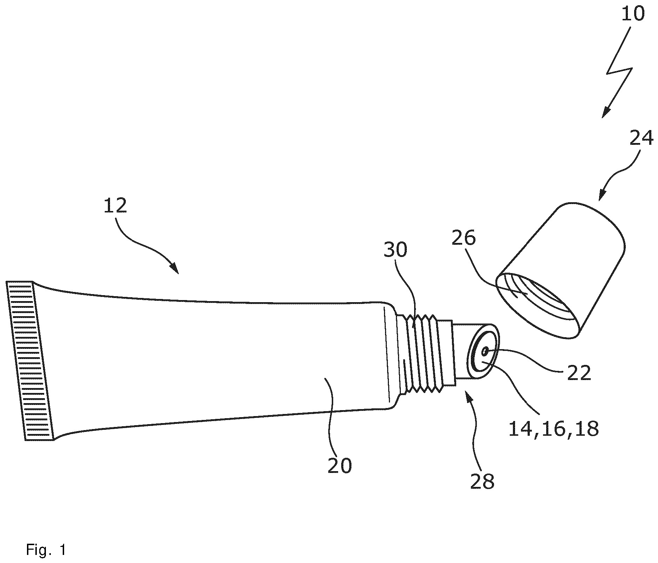

- Fig. 1: zeigt eine isometrische Ansicht eines erfindungsgemäßen Dermabrasionswerkzeugs.

**Fig. 1** zeigt ein Dermabrasionswerkzeug **10** mit einem Handteil **12** und einem Dermabrasionsbereich **14,** wobei der Dermabrasionsbereich 14 eine Trägermatrix **16** mit mehreren Reibvorsprüngen **18** zur mechanischen Hautbehandlung aufweist.

Das Handteil 12 ist elastisch und/oder tubenförmig ausgebildet, sodass durch Pressen, Drehen und/oder Drücken des Handteils 12 ein Fluid **20** im Inneren des Handteils 12 durch eine Öffnung **22** aus dem Handteil 12 pressbar ist.

Das Dermabrasionswerkzeug 10 weist einen Deckel **24** in Form eines Schraubdeckels mit einem Deckelgewinde **26** auf. Das Deckelgewinde 26 ist vorliegend in Form eines Innengewindes ausgebildet. Das Handteil 12 weist einen Gewindevorsprung **28** mit einem Handteilgewinde **30** auf. Das Handteilgewinde 30 ist vorliegend in Form eines Außengewindes ausgebildet.

Die Trägermatrix 16 ist vorliegend in Form einer flachen Scheibe ausgebildet, die stirnseitig am Gewindevorsprung 28 angeordnet oder ausgebildet ist. Die Trägermatrix 16 umringt im vorliegenden Fall die Öffnung 22.

Unter Betrachtung der Zeichnung betrifft die Erfindung zusammenfassend ein mit Fluid 20 gefülltes Dermabrasionswerkzeug 10 mit einer Öffnung 22 zum Austritt des Fluids 20. Ein Handteil 12 ist vorzugsweise zumindest teilweise containerartig oder tubenartig ausgebildet. Ein Dermabrasionsbereich 14 ist bevorzugt im Bereich der Öffnung 22 angeordnet oder ausgebildet, kann aber auch auf einer anderen Stelle des Dermabrasionsbereiches 14 liegen.

### Bezuaszeichenliste

- 10: Dermabrasionswerkzeug
- 12: Handteil
- 14: Dermabrasionsbereich
- 16: Trägermatrix
- 18: Reibvorsprünge
- 20: Fluid
- 22: Öffnung
- 24: Deckel
- 26: Deckelgewinde
- 28: Gewindevorsprung
- 30: Handteilgewinde

## Patentansprüche

1. Dermabrasionswerkzeug (10) mit einem Handteil (12) zum manuellen Halten und Führen des Dermabrasionswerkzeugs (10) und einem Dermabrasionsbereich (14) am axialen Ende des Dermabrasionswerkzeugs (10), wobei der Dermabrasionsbereich (14) eine Trägermatrix (16) mit mehreren Reibvorsprüngen (18) zur mechanischen Hautbehandlung aufweist, die an der Trägermatrix (16) angeordnet oder in der Trägermatrix (16) ausgebildet sind, wobei die Reibvorsprünge (18) jeweils eine maximale Querschnittsbreite zwischen 0,5 µm und 1000 µm aufweisen, wobei das Handteil (12) einen mit einem Fluid (20) gefüllten Hohlraum aufweist, wobei das Dermabrasionswerkzeug (10) eine mit dem Hohlraum fluidisch verbundene Öffnung (22) aufweist,
**dadurch gekennzeichnet, dass**
das Handteil (12) zumindest abschnittsweise elastisch und/oder plastisch verformbar ausgebildet ist, sodass ein Nutzer durch manuelles Pressen und/oder Drehen des Handteils (12) das Fluid (20) aus der Öffnung (22) pressen kann.

2. Dermabrasionswerkzeug (10) nach Anspruch 1, bei dem das Handteil (12) eine Kunststofffolie aufweist, sodass es elastisch und/oder plastisch verformbar ausgebildet ist.

3. Dermabrasionswerkzeug (10) nach einem der vorhergehenden Ansprüche, bei dem das Handteil (12) überwiegend elastisch ausgebildet ist

4. Dermabrasionswerkzeug (10) nach einem der vorhergehenden Ansprüche, bei dem die Öffnung (22) im Dermabrasionsbereich (14) ausgebildet ist.

5. Dermabrasionswerkzeug (10) nach einem der vorhergehenden Ansprüche, bei dem das Handteil (12) in Form einer Tube oder eines Containers ausgebildet ist.

6. Dermabrasionswerkzeug (10) nach einem der vorhergehenden Ansprüche, bei dem das Dermabrasionswerkzeug (10) einen Deckel (24) zum Verschließen der Öffnung (22) aufweist.

7. Dermabrasionswerkzeug (10) nach Anspruch 6, bei dem der Deckel (24) in Form eines Schraubdeckels mit einem Deckelgewinde (26) ausgebildet ist, wobei das Handteil (12) einen axialen Gewindevorsprung (28) mit einem zum Deckelgewinde (26) passenden Handteilgewinde (30) aufweist, wobei der Gewindevorsprung (28) die Öffnung (22) aufweist.

8. Dermabrasionswerkzeug (10) nach Anspruch 7, bei dem der Gewindevorsprung (28) den Dermabrasionsbereich (14) aufweist.

9. Dermabrasionswerkzeug (10) nach einem der vorhergehenden Ansprüche, bei dem die Trägermatrix (16) in Form einer flachen Scheibe ausgebildet ist.

10. Dermabrasionswerkzeug (10) nach einem der vorhergehenden Ansprüche, bei dem die Reibvorsprünge (18) in Form von Partikeln ausgebildet sind, die in die Trägermatrix (16) eingebettet sind.
